# EUROPÄISCHE PATENTANMELDUNG

(11) **EP 4 524 550 A1**
(43) Veröffentlichungstag der Anmeldung: **19.03.2025**
(21) Anmeldenummer: 24201136.9
(22) Anmeldetag: 18.09.2024
(51) Int. Cl.: G01N 21/3554, G01N 21/3563, G01N 21/90, G01N 21/3581, G01N 21/359

(54) **VERFAHREN UND VORRICHTUNG ZUM BESTIMMEN VON EIGENSCHAFTEN VON AUS PAPIER ODER PAPPE HERGESTELLTEN BEHÄLTNISSEN**

(30) Priorität: 18.09.2023 DE 102023125194
(71) Anmelder: Krones AG, 93073 Neutraubling (DE)
(72) Erfinder: Niedermeier, Anton, 93073 Neutraubling (DE); Piana, Stefan, 93073 Neutraubling (DE)
(74) Vertreter: Bittner, Bernhard

(57) **Zusammenfassung**

Verfahren zum Bestimmen von Behältniseigenschaften, wobei ein zu untersuchendes Behältnis (10) entlang eines vorgegebenen Transportpfads transportiert wird und dieses Behältnis (10) einen Bodenbereich (10a), einen Grundkörper (10b) der zu Aufnahme eines Füllguts dient und einen Mündungsbereich aufweist und wobei dieses Behältnis wenigstens abschnittsweise aus einem Papier- oder Pappe enthaltenden Material besteht, dadurch gekennzeichnet, dass eine Strahlungseinrichtung (42) wenigstens einen Abschnitt des Behältnisses mit elektromagnetischer Strahlung bestrahlt und eine Sensoreinrichtung (44) von dem Behältnis in Reaktion auf die eingestrahlte Strahlung reflektierte und/oder transmittierte und/oder ausgestrahlte Strahlung erfasst und aus dieser Strahlung wenigstens eine für das Behältnis (10) charakteristische Größe ermittelt wird.

## Beschreibung

Die vorliegende Erfindung bezieht sich auf ein Verfahren und eine Vorrichtung zum Bestimmen von Behältniseigenschaften. Derartige Verfahren und Vorrichtungen sind aus dem Stand der Technik seit langem bekannt. So ist es beispielsweise bekannt, dass bei der Herstellung von Kunststoffbehältnissen Eigenschaften derselben ermittelt werden, wie beispielsweise eine Wandstärke des Behältnisses oder dergleichen. Diese Eigenschaften sind bedeutsam, um hieraus auf eine Qualität des gefertigten Behältnisses zu schließen, beispielsweise darauf, ob die ermittelte Wandstärke innerhalb eines Sollbereichs liegt.

Bei diesen Verfahren werden üblicherweise optische Messeinrichtungen verwendet, beispielsweise wird das Behältnis mit Licht durchleuchtet und eine Transmission bestimmt, aus der sich wiederum auf die Behältniswandstärke rückschließen lässt.

In jüngerer Zeit ist man vermehrt bestrebt, umweltfreundliche Alternativen für Behältnisse herzustellen. Ein Ansatz ist darauf gerichtet, Behältnisse aus Pulpe, insbesondere aus Papier- oder Papppulpe herzustellen. Die ersten Versuche sind vielversprechend. Für derartige Behältnisse tritt jedoch der Nachteil auf, dass diese in der Regel nicht mit sichtbarem Licht inspiziert werden können, weil sie dieses nicht transmittieren.

Im Stand der Technik sind bereits Getränkebehälter in Form von Pappmachebehältern, Pulpebehältern, Fasergussbehältern und dergleichen bekannt, welche üblicherweise in mehreren Prozessschritten aus einer wässrigen Mache hergestellt werden. Nach einer ausreichenden Trocknung kann diese Art von Behältern befüllt und verschlossen werden. Es ist aber weiterhin bekannt, dass Pulpe bei der Trocknung schrumpft. Auf diese Weise kann eine Überprüfung etwa der Maßhaltigkeit erst nach einer ausreichenden Trocknung durchgeführt werden.

Ein weiteres Problem derartiger Behältnisse ist, dass diese oftmals noch eine Restfeuchte aufweisen. Im Stand der Technik ist es möglich, eine Restfeuchtebestimmung bei einer Kartonqualitätskontrolle durchzuführen, beispielsweise über ein Hygrometer. Diese Messung ist dabei berührend mit einem Schwertfühler. Ein Schwertfühler muss mit dem Material in Kontakt sein, bis sich ein ausreichend stabiler Messwert einstellt.

Diese Verfahren eignen sich jedoch nicht für die Untersuchung von den hier relevanten Behältnissen. Insbesondere die berührende Messung würde sich stark auf die Leistung entsprechender Maschinen auswirken.

Wie erwähnt, ist nach der Produktion von derartigen Pulpebehältnissen und damit üblicherweise vor der Weiterverarbeitung (einem Befüllen, Verschließen und dergleichen) noch eine Restfeuchte in der Behältniswandung enthalten. Diese sollte einen definierten Wert nicht überschreiten, damit das Behältnis in der Anlage, bei der Lagerung und dergleichen vernünftig handhabbar ist (insbesondere im Hinblick auf Stabilität, Abriebfestigkeit, Schutz vor Schimmel und dergleichen). Für die Bestimmung der Restfeuchte existieren, wie oben erwähnt, verschiedene etablierte Verfahren oder Ideen wie das Wiegen, eine radiometrische Absorptionsmessung und dergleichen. Diese Verfahren sind jedoch entweder langsam, aufwendig oder teuer oder können nicht exakt unterscheiden zwischen Wandstärkeunterschieden und unterschiedlichen Restfeuchtegehalten.

Der vorliegenden Erfindung liegt daher die Aufgabe zugrunde, ein Bestimmungsverfahren und auch eine Bestimmungsvorrichtung zur Verfügung zu stellen, welche insbesondere für die hier beschriebenen Behältnisse, die aus Pulpe hergestellt sind, angewandt werden können. Diese Aufgaben werden erfindungsgemäß durch die Gegenstände der unabhängigen Patentansprüche gelöst. Vorteilhafte Ausführungsformen und Weiterbildungen sind Gegenstand der Unteransprüche.

Bei einem erfindungsgemäßen Verfahren zum Bestimmen von Behältniseigenschaften wird ein zu untersuchendes Behältnis entlang eines vorgegebenen Transportpfads transportiert und dieses Behältnis weist einen Bodenbereich, einen Grundkörper, der zur Aufnahme des Füllguts dient und einen Mündungsbereich auf. Weiterhin ist dieses Behältnis wenigstens abschnittsweise oder lagenweise und bevorzugt im Wesentlichen und besonders bevorzugt vollständig aus einem Papier oder Pappe enthaltenden Material hergestellt und/oder besteht aus einem solchen Material.

Erfindungsgemäß bestrahlt eine Strahlungseinrichtung wenigstens einen Abschnitt des Behältnisses mit elektromagnetischer Strahlung und/oder strahlt auf wenigstens einen Abschnitt des Behältnisses elektromagnetische Strahlung ein.

Weiterhin erfasst eine Sensoreinrichtung von dem Behältnis in Reaktion auf die eingestrahlte Strahlung reflektierte und/oder transmittierte und/oder ausgestrahlte Strahlung und es wird aus dieser Strahlung wenigstens eine für das Behältnis charakteristische Größe ermittelt.

Bei den hier beschriebenen erfindungsgemäßen Vorgehensweisen wird jeweils das Behältnis selbst oder eine Wandung oder ein Abschnitt der Wandung untersucht um daraus einen hierfür charakteristischen Wert, beispielsweise eine Restfeuchte oder eine Wandungsdicke abzuleiten. Bevorzugt weist das Material des Behältnisses einen vorbestimmten Flüssigkeit- und insbesondere Wasseranteil auf.

Unter in Reaktion auf die eingestrahlte Strahlung kann verstanden werden, dass die von dem Behältnis transmittierte Strahlung aufgenommen und/oder analysiert und/oder untersucht wird. Es wäre jedoch auch möglich, dass eine von dem Behältnis reflektierte Strahlung aufgenommen, und/oder analysiert und/oder untersucht wird. Daneben ist es auch möglich, dass infolge der eingestrahlten Strahlung Effekte an dem Behältnis auftreten, insbesondere (lokale) Erwärmungen und wiederum diese Erwärmungen gemessen werden, um so auf die Eigenschaft des Behältnisses rückzuschließen. Eine weitere mögliche Reaktion wäre das Aussenden von Fluoreszenz- bzw. Phosphoreszenzlicht.

Insbesondere auch durch den besagten Wasseranteil und/oder die Restfeuchte unterscheidet sich das Behältnis von aus dem Stand der Technik bekannten Behältnissen, welche insbesondere aus Glas oder Kunststoff hergestellt ist. Die Anmelderin hat daher Lösungen entwickelt, welche insbesondere zur Anwendung mit wasserenthaltenden Materialien geeignet sind und/oder welche zur Anwendung mit Papier und/oder Pappmaterialien geeignet sind.

Bei einer bevorzugten Ausführungsform besteht das Behältnis aus Papier und/oder Pappe und bevorzugt aus einer papier- oder pappehaltigen Pulpe.

Bevorzugt weist eine Mündung des Behältnisses einen kleineren Querschnitt auf als derjenige des Grundkörpers des Behältnisses.

Bei einem weiteren bevorzugten Verfahren ist die eingestrahlte Strahlung aus einer Gruppe von elektromagnetischen Strahlungen ausgewählt, welche ultraviolette Strahlung, Strahlung im sichtbaren Wellenlängenbereich, nahinfrarote Strahlung, ferninfrarote Strahlung, allgemein infrarote Strahlung, Wärmestrahlung, Terahertzstrahlung, Mikrowellenstrahlung, Radiowellen, Röntgenstrahlung, radioaktive Strahlung und dergleichen enthält.

Für die weitere oben im Rahmen der Erfindung beschriebene Vorgehensweise sind mehrere Möglichkeiten denkbar. So ist beispielsweise eine thermische Restfeuchtemessung möglich. Dabei kann ein Wärmeeintrag insbesondere ein definierter Wärmeeintrag in das Behältnis vorgenommen werden. Dies kann beispielsweise über Wärmestrahlung beim Durchlauf durch einen Strahlungsofen erfolgen. Bevorzugt wird nach einer vorgegebenen Zeit und insbesondere einer exakt definierten Zeit eine Temperaturmessung vor und nach dem Wärmeeintrag vorgenommen. Eine hohe Feuchte bedeutet eine hohe Wärmekapazität und führt zu einer vergleichsweise geringen Erwärmung.

Eine niedrige Feuchte führt zu einer großen Erwärmung, da die spezifische Wärmekapazitive der Pulpe, d. h. des Materials des Behältnisses sehr viel niedriger ist. Auf diese Weise kann ein Rückschluss von der Höhe des Temperatursprungs auf die Restfeuchte durchgeführt werden. Wie unten genauer erläutert, ist hier auch eine Überwachung und auch eine Closed-Loop (geschlossener Regelkreis) Regelung des Trocknungsvorgangs möglich.

Bevorzugt erfolgt daher in einer bevorzugten Ausführungsform durch die erste Strahlungseinrichtung ein Wärmeeintrag.

Bevorzugt ermittelt eine Erfassungseinrichtung eine (lokale) Temperatur des Behältnisses und/oder einer Wandung des Behältnisses.

Besonders bevorzugt erfolgt hier der Wärmeeintrag und die Temperaturbestimmung zeitlich versetzt. Besonders bevorzugt erfolgt die Temperaturbestimmung innerhalb eines vorgegebenen Zeitraums nach dem Temperatureintrag. Besonders bevorzugt ist dieser Zeitraum geringer als 20 Sekunden, bevorzugt geringer als 10 Sekunden, bevorzugt geringer als 5 Sekunden, bevorzugt geringer als 3 Sekunden, bevorzugt geringer als 2 Sekunden und bevorzugt geringer als 1 Sekunde.

Bei einem weiteren bevorzugten Verfahren werden wenigstens zwei und bevorzugt mehrere Temperaturmessungen durchgeführt. Bevorzugt werden diese Temperaturmessungen zu vorbestimmten Zeitpunkten und/oder vorbestimmten Zeiträumen durchgeführt.

Werden mehrere Temperaturmessungen in einem genau definierten Zeitraster durchgeführt, dann kann aus den so gewonnenen Stützwerten der Abklingkurve nicht nur auf die Restfeuchte, sondern darüber hinaus auch auf die Wandstärke geschlossen werden.

Dies beruht darauf, dass die Abkühlung der Außenfläche sowohl durch Wärmeabstrahlung nach außen als auch durch Wärmeleitung nach innen in die Wandung erfolgt (der Anteil der Konvektion ist bei kurzen Messzeiten sehr gering und kann vernachlässigt werden). Mit grö-βeren Wandstärken steigt der Anteil der Wärmeleitung, so dass die Außentemperatur schneller abnimmt, die Abklingzeitkonstante also kürzer ist.

Besonders bevorzugt erfolgt auch eine Temperaturbestimmung der Behältnisse vor dem Wärmeeintrag. So kann beispielsweise vor dem Durchlauf durch einen Ofen die Höhe des Temperatursprungs bestimmt werden.

Besonders bevorzugt erfolgt sowohl der Temperatureintrag als auch die Bestimmung der Temperatur mittels der Erfassungseinrichtung während der Bewegung der Behältnisse. Bevorzugt erfolgt die Temperaturbestimmung berührungslos, z.B. mittels Thermopile-Sensor oder Wärmebildkamera, bevorzugt im Wellenlängenbereich zwischen 7 und 14 µm.

Bei einem weiteren bevorzugten Verfahren handelt es sich bei der Strahlung um infrarote Strahlung einen Wellenlängenbereich zwischen 800nm und 2500nm, bevorzugt zwischen 900nm und 2200, bevorzugt zwischen 1000nm und 2000nm und besonderes bevorzugt zwischen 1200 nm und 1800 nm oder es handelt sich bei der Strahlung um Wärmestrahlung, welche einen definierten Wärmeeintrag in wenigstens einem Abschnitt des Behältnisses bewirkt.

Besonders bevorzugt handelt sich bei diesem Verfahren um ein Durchlichtverfahren, bei dem die Strahlung durch das Behältnis tritt und von der Erfassungseinrichtung aufgenommen wird. Bei diesem Verfahren werden bevorzugt beide Vorder- und Hinterwandungen des Behältnisses gleichzeitig mit infrarotem Licht durchstrahlt. Die Wellenlängen des SWIR (kurzwelliges Infrarotlicht) Lichts sind, wie oben erwähnt in dem geeigneten Spektralbereich zwischen 1200 nm und 1800 nm. Dieser Spektralbereich ist entsprechend so gewählt, dass Wasser relativ stark absorbiert und die Durchdringung durch die Pulpe und/oder das Material des Behältnisses stark genug ist.

Bei einem weiteren bevorzugten Verfahren wird eine Messung in wenigstens zwei Spektralbereichen und insbesondere zwei Spektralbereichen mit unterschiedlich starker Absorption vorgenommen. Auf diese Weise können Umwelteinflüsse leichter kompensiert werden.

Bei einem weiteren bevorzugten Verfahren wird eine für das Behältnis charakteristische Größe in der Längsrichtung des Behältnisses abschnittsweise ermittelt und/oder die wenigstens eine für das Behältnis charakteristische Größe wird in Abhängigkeit der Längsrichtung des Behältnisses bestimmt.

Auf diese Weise wird eine abschnittsweise Messung oder Vermessung des Behältnisses in Abhängigkeit von dessen Längsrichtung vorgenommen.

Bei einem weiteren bevorzugten Verfahren erfolgt die Messung der charakteristischen Größe während einer Bewegung der Behältnisse. Es wird darauf hingewiesen, dass diese bevorzugte Variante für alle die hier vorgeschlagenen Verfahren möglich ist.

Bei einem weiteren bevorzugten Verfahren ist die für das Behältnis charakteristische Größe ein Feuchtewert und insbesondere eine Restfeuchte des Behältnisses und/oder eine Wanddicke wenigstens eines Wandungsabschnitts des Behältnisses.

Bei einem weiteren bevorzugten Verfahren wird das Behältnis während der Messung gedreht und insbesondere bezüglich seiner Längsrichtung gedreht. Auf diese Weise ist eine Vermessung der gesamten Mantelfläche des Behältnisses möglich. Es wird darauf hingewiesen, dass diese bevorzugte Ausführungsform für die oben beschriebenen Messungen mittels elektromagnetischer Strahlung geeignet ist.

Bei einem weiteren bevorzugten Verfahren ist die Sensoreinrichtung dazu bestimmt, ein ortsaufgelöstes Bild der von den Behältnis reflektierten und/oder transmittierten und/oder ausgestrahlten Strahlung aufzunehmen. In anderen Worten nimmt bevorzugt die Sensoreinrichtung ein ortsaufgelöstes Bild der von dem Behältnis reflektierten und/oder transmittierten und/oder ausgestrahlten Strahlung auf.

Bei einer bevorzugten Ausführungsform handelt es sich bei der Bildaufnahmeeinrichtung um eine ortsauflösende Kamera (diese kann sowohl als Matrix als auch als Zeilenkamera ausgeführt sein). Besonders bevorzugt, kann diese Bildaufnahmeeinrichtung bzw. Kamera Bereiche mit zu hoher Restfeuchte als Abdunklung erkennen.

Daneben wäre es möglich, einen integrierenden Flächensensor zu verwenden, der ein Restfeuchtemaß für das gesamte Behältnis anhand der Absorption misst.

Insbesondere für eine Messung mittels infrarotem Licht wird bevorzugt eine ortsaufgelöste NIR- oder SWIR-Kamera eingesetzt. Auch hierbei kann es sich sowohl eine Matrix- als auch um eine Zeilenkamera handeln. Diese Bildaufnahmeeinrichtung und/oder Kamera kann Bereiche mit zu hoher Restfeuchte als Abdunklung erkennen.

Alternativ kann in diesem Fall auch als Sensoreinrichtung ein Array aus Einzelsensoren verwendet werden. Daneben wäre es auch hier möglich, einen integrierenden Flächensensor zu verwenden, der ein Restfeuchtemaß für das gesamte Behältnis anhand der Absorption misst oder bestimmt.

Bei einem weiteren bevorzugten Verfahren befindet sich das Behältnis zum Zwecke der Bestimmung der Behältniseigenschaften wenigstens zeitweise zwischen einer oder der Strahlungseinrichtung und der Sensoreinrichtung bzw. wird dort angeordnet.

Dabei ist es möglich, dass sich der Transportpfad zwischen der Strahlungseinrichtung und der Sensoreinrichtung erstreckt. In diesem Falle wird beispielsweise das Behältnis zwischen der Strahlungseinrichtung und der Sensoreinrichtung hindurch bewegt. In diesem Falle verläuft bevorzugt ein optischer Pfad zwischen der Strahlungseinrichtung und der Sensoreinrichtung durch das Behältnis hindurch und/oder verläuft quer zu dem Transportpfad der Behältnisse. Dabei kann dieser optischer Pfad senkrecht zu dem Transportpfad stehen oder auch schräg dazu.

Insbesondere schneidet jedoch der optische Pfad den Transportpfad des Behältnisses. In einem anderen Fall wird bevorzugt das Behältnis von der Strahlungseinrichtung zu der Sensoreinrichtung transportiert und befindet sich daher entlang des Transportpfads zeitweise zwischen der Strahlungseinrichtung und der Sensoreinrichtung. Bevorzugt befindet sich das Behältnis wenigstens zeitweise zwischen einer vertikalen und senkrecht zu dem Transportpfad der Behältnisse stehenden Ebene, welche durch die Strahlungseinrichtung verläuft und einer vertikalen und senkrecht zu dem Transportpfad der Behältnisse stehenden Ebene, welche durch die Sensoreinrichtung verläuft.

Während in dem oben genannten ersten Fall eine Messung gerade zu dem Zeitpunkt oder dem Zeitraum erfolgt, indem sich das Behältnis zwischen der Strahlungseinrichtung und der Sensoreinrichtung befindet, findet in dem zweiten Fall in dem Zeitraum, in dem sich das Behältnis zwischen der Strahlungseinrichtung und der Sensoreinrichtung befindet, bevorzugt keine Messung statt.

Bei einem weiteren bevorzugten Verfahren findet die Einstrahlung von Licht auf das Behältnis und die Messung mit der Sensoreinrichtung im Wesentlichen gleichzeitig statt. Unter im Wesentlichen gleichzeitig wird auch verstanden, dass die Laufzeit von Licht oder Strahlung unberücksichtigt bleibt und daher die Einstrahlung von Licht und die Aufnahme durch die Sensoreinrichtung im Wesentlichen nur um die Zeit zueinander versetzt sind, welche die Strahlung von der Strahlungseinrichtung zu der Sensoreinrichtung benötigt.

Bei der zweiten genannten Ausführungsform finden bewusst die Einstrahlung und die Messung zeitversetzt statt, wie oben angemerkt. Die Sensoreinrichtung bestimmt in diesem Fall insbesondere eine Temperatur und/oder eine Temperaturdifferenz des erwärmten Bereiches und/oder des erwärmten Behältnisses.

Besonders bevorzugt wird aus einer bekannten oder einer konstanten Restfeuchte eine Wanddicke des Behältnisses bestimmt. Hierbei ist zu berücksichtigen, dass in die Berechnung der Restfeuchte auch die Wanddicke eingeht. Ist die Restfeuchte bekannt oder konstant, kann aus den Messungen auch die Wandstärke ermittelt werden.

Bei einer bevorzugt durchgeführten Drehung des Behältnisses (beispielsweise durch Abrollen) ist eine Messung der gesamten Mantelfläche möglich.

Die oben genannte thermische Messung hat den Vorteil, dass thermische Strahler und Langwellen-Infrarot- und/oder Flächensensoren vergleichsweise preisgünstig sind, sodass sich ein Messsystem zu einem überschaubaren Preis realisieren lässt.

Die Messung der Wandungstemperaturen kann mit mehreren einzelnen solcher Sensoren durchgeführt werden. Bei der Anordnung mehrerer Sensoren, beispielsweise als vertikales Sensor-Array kann ein vertikales Restfeuchteprofil bestimmt werden.

Bei mehreren Messungen von verschiedenen Seiten, beispielsweise in einem 90° Winkelraster und/oder bei mehrfacher Messung (beispielsweise zeitlich kurz hintereinander) kann die Mantelfläche (zumindest grob) komplett erfasst werden. Auf diese Weise kann auch eine ungleichmäßige Verteilung der Restfeuchte messtechnisch erfasst werden.

Alternativ können zu diesem Zwecke der Messung eine oder mehrere thermische Infrarotkameras eingesetzt werden.

Bei kurzen Bestrahlungszeiten, bevorzugt in einem Bereich zwischen 10 ms und 1000 ms, bevorzugt zwischen 50 ms und 500 ms und besonders bevorzugt zwischen 70 ms und 400 ms und besonders bevorzugt zwischen 70 ms und 200 ms und bevorzugt kurzen Zeitdifferenzen zwischen der Bestrahlung und der Messung geht die Wandstärke praktisch nicht in das Messergebnis ein und es kann der Feuchtegehalt an der Oberfläche der Wandung recht genau ermittelt werden.

Besonders bevorzugt wird daher mit Bestrahlungszeiten gemessen, die in den oben genannten Bereichen liegen.

Bevorzugt wird eine Bestrahlung verwendet, die in einem Wellenlängenbereich liegt, der von Wasser stark oder sehr stark absorbiert wird. Besonders bevorzugt werden Bestrahlungen in einem Wellenlängenbereich von 1 µm bis 4 µm, bevorzugt 1,2 µm bis 3 µm und besonders bevorzugt von 1,5 µm und 2,5 µm Wellenlänge verwendet.

Bei einem weiteren bevorzugten Verfahren erfolgt die Bestimmung der genannten Eigenschaften und/oder die Messung berührungslos. Die genannten Verfahren haben den Vorteil, dass sie nicht zu einer Begrenzung der Produktionsleistung führen. Bevorzugt kann bei einigen der genannten Verfahren, insbesondere der Messung mittels elektromagnetischer Strahlung auf zwei Spektralbereichen mit unterschiedlich starker Absorption gemessen werden und auf diese Weise können beispielsweise Materialdickenschwankungen, Messschwankungen und Umwelteinflüsse kompensiert werden.

Sämtliche der genannten Verfahren erlauben eine Qualitätskontrolle des produzierten Behältnisses. Diese gemessenen Werte können auch verwendet werden, um eine anschließende Trocknung der Behältnisse durchzuführen. Auf diese Weise kann auch eine Closed-Loop-Regelung beispielsweise des Materialeinsatzes, eines Materialmischungsverhältnisses, eines Wasseranteils, einer Trocknungsenergie, einer Trocknungszeit und dergleichen vorgenommen werden.

Bevorzugt werden die hier beschriebenen Messungen an einem unbefüllten Behältnis durchgeführt. Bevorzugt werden die hier beschriebenen Messungen an einem unverschlossenen Behältnis durchgeführt.

Bevorzugt werden die hier beschriebenen Messungen an einem erstmalig zu befüllenden Behältnis durchgeführt.

Die vorliegende Erfindung ist weiterhin auf eine Vorrichtung zum Bestimmen von Behältniseigenschaften gerichtet. Diese weist eine Transporteinrichtung auf, welche ein zu untersuchendes Behältnis (und bevorzugt eine Vielzahl von zu untersuchenden Behältnissen) entlang eines vorgegebenen Transportpfads transportiert, wobei dieses Behältnis einen Bodenbereich, einen Grundkörper, der zur Aufnahme eines Füllguts dient und einen Mündungsbereich aufweist und wobei dieses Behältnis wenigstens abschnittsweise und bevorzugt vollständig aus einem Papier oder Pappe enthaltenen Material besteht.

Erfindungsgemäß weist die Vorrichtung eine Strahlungseinrichtung auf, welche auf wenigstens einen Abschnitt des Behältnisses elektromagnetische Strahlung einstrahlt und eine Sensoreinrichtung, welche von dem Behältnis in Reaktion auf die eingestrahlte Strahlung reflektierte und/oder transmittierte und/oder ausgestrahlte Strahlung erfasst. Weiterhin ist eine Auswerteeinrichtung vorgesehen, welche aus dieser Strahlung wenigstens eine für das Behältnis charakteristische Größe ermittelt.

Es wird daher auch vorrichtungsseitig vorgeschlagen, dass eine Messung des Behältnisses berührungslos erfolgt. Bevorzugt erfolgt die Messung auch Inline, d. h. während eines Herstellungsbetriebs.

Zur Messung der jeweiligen Größen werden wie oben erwähnt, insbesondere elektromagnetische Strahlung und/oder kapazitive Messungen eingesetzt.

Bevorzugt handelt sich bei der für das Behältnis charakteristischen Größe um eine Feuchte und/oder einen Feuchtegehalt und/oder eine Wanddicke, insbesondere eine Wanddicke in einem Bereich des Bodens oder des Grundkörpers des Behältnisses.

Besonders bevorzugt, handelt es sich bei der Transporteinrichtung um ein Transportband oder eine Transportkette. Besonders bevorzugt erfolgt der Transport der Behältnisse entlang einer geradlinigen Richtung und insbesondere in einem stehenden Zustand.

Es wäre jedoch auch möglich, dass die Transporteinrichtung einen drehbaren Träger aufweist, an dem eine Vielzahl von Halteelementen zum Halten der Behältnisse angeordnet ist.

Bei der bevorzugten Ausführungsform weist die Sensoreinrichtung eine Bildaufnahmeeinrichtung auf, welche zur Aufnahme eines ortsaufgelösten Bildes wenigstens eines Abschnitts des Behältnisses ausgebildet und angeordnet ist. Besonders bevorzugt ist die Bildaufnahmeeinrichtung aus einer Gruppe von Bildaufnahmeeinrichtungen ausgewählt, welche Röntgenkameras, insbesondere Röntgenzeilen oder Matrixkameras, ortsauflösende Kameras, insbesondere ortsauflösende Nah-Infrarotkameras, ortsauflösende SWIR-Kameras, ortsauflösende LWIR-Kameras, thermische IR Kameras, Arrays und Einzelsensoren und dergleichen enthält.

Diese Ausgestaltung ist insbesondere für all diejenigen Varianten anwendbar, welche Messungen mittels elektromagnetischer Strahlung durchführen.

Bei einer weiteren bevorzugten Ausführungsform weist die Vorrichtung eine Dreheinrichtung auf, welche dazu geeignet und bestimmt ist, die Behältnisse insbesondere bezüglich ihrer Längsrichtung zu drehen.

Dabei es ist in einer Variante möglich, dass diese Drehung erfolgt, wenn die Messung durchgeführt wird, es wäre jedoch auch möglich, dass mehrfach abschnittsweise entlang des Transportpfads gedreht und auch gemessen wird.

Bevorzugt kann die Dreheinrichtung beispielsweise Seitenführungsbänder aufweisen, welche an den Grundkörper der Behältnisse anlegbar sind und so auch dessen Drehung bewirken.

Die vorliegende Erfindung ist weiterhin auf eine Anordnung zum Behandeln von Behältnissen mit einer Vorrichtung der oben beschriebenen Art gerichtet sowie einer Trocknungseinrichtung zum Trocknen der Behältnisse, wobei bevorzugt diese Trocknungseinrichtung in Abhängigkeit von der für das Behältnis charakteristischen Größe, insbesondere einer Restfeuchte steuerbar ist.

Bevorzugt ist diese Trocknungseinrichtung nach der oben beschriebenen Vorrichtung angeordnet. Besonders bevorzugt erlaubt die Steuerungseinrichtung eine Regelung und insbesondere eine Regelung in Form eines Closed-Loops bzw. eines geschlossenen Regelkreises (bevorzugt ist in diesem Fall die Sensoreinrichtung nach der Behandlungseinrichtung angeordnet).

So kann beispielsweise eine Heizleistung der Trocknungseinrichtung geändert werden. Daneben oder zusätzlich kann auch die Heizdauer (etwa über die Transportgeschwindigkeit verändert werden. Weiterhin kann auch die Stärke der Konvektionsströmung und/oder deren örtliche Verteilung (um lokale Feuchtenester zu vermeiden) geändert werden. Auch kann der Feuchtegrad der Trockungsluft verändert werden.

Weiterhin kann die Anordnung mehrere der oben beschriebenen Vorrichtungen aufweisen.

So ist es möglich, dass beispielsweise hintereinander entlang des Transportpfades unterschiedliche Messungen durchgeführt werden, beispielsweise eine Messung mittels nah-infrarotem Licht sowie eine kapazitive Messung.

Die Messergebnisse können dabei beispielsweise redundant verwendet werden, es wäre doch auch möglich, dass mit einer ersten Messung eine Restfeuchte und mit einer weiteren Messung eine Wanddickenbestimmung vorgenommen wird. Der Fachmann erkennt, dass alle der hier vorgeschlagenen Vorrichtungen miteinander kombiniert werden können, beispielsweise eine Messung mit Röntgenstrahlung mit einer Messung mit infrarotem Licht oder auch eine kapazitive Messung mit einer Wärmemessung oder weitere Ausgestaltungen.

Weitere Vorteile und Ausführungsformen ergeben sich aus den beigefügten Zeichnungen: Darin zeigen:
- Fig. 1: eine schematische Darstellung einer erfindungsgemäßen Vorrichtung;
- Fig. 2: eine schematische Darstellung einer Messung mit nah-infrarotem Licht;
- Fig. 3: eine Messung mittels Erwärmung; und
- Fig. 4: eine weitere Darstellung einer erfindungsgemäßen Vorrichtung.

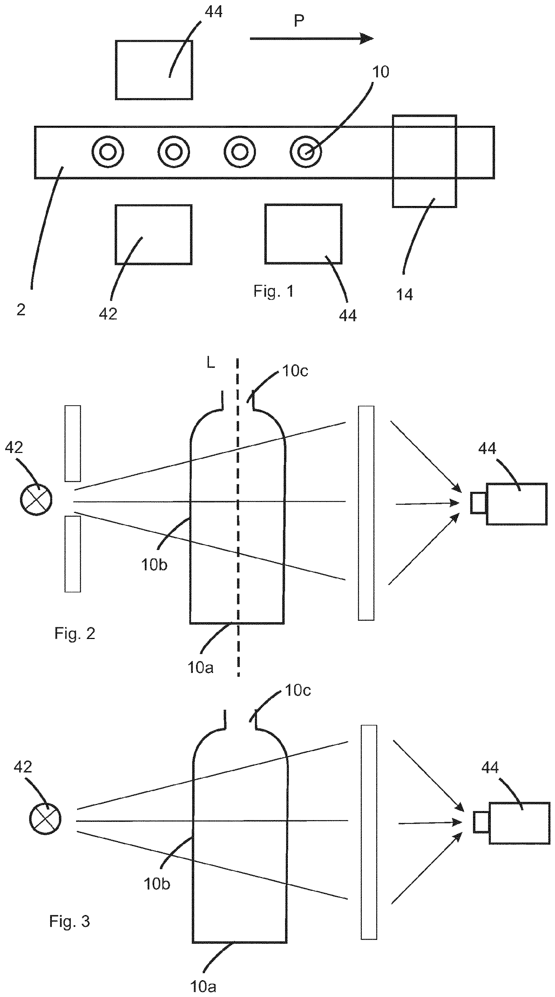

Fig. 1 zeigt eine grobschematische Darstellung einer erfindungsgemäßen Vorrichtung 1. Dabei ist eine Transporteinrichtung 2, wie etwa ein Transportband vorgesehen, welches Behältnisse 10 entlang eines Transportpfads P transportiert. Das Bezugszeichen 42 kennzeichnet eine Strahlungseinrichtung, welche Strahlung auf die Behältnisse 10 einstrahlt und das Bezugszeichen 44 eine Sensoreinrichtung, welche die von den Behältnissen transmittierte oder reflektierte Strahlung aufnimmt. Alternativ wäre auch die Anordnung der rechts gezeigten Sensoreinrichtung 44 möglich, die beispielsweise eine durch die Strahlungseinrichtung 42 bewirkte Erwärmung feststellt.

Das Bezugszeichen 14 kennzeichnet eine Trocknungseinrichtung, welche zum Trocknen der Behältnisse 10 dient. Bevorzugt ist eine Steuerungseinrichtung zum Steuern dieser Trocknungseinrichtung vorgesehen, welche diesen Trocknungsvorgang insbesondere in Abhängigkeit von einem von der Sensoreinrichtung festgestellten Messwert wie insbesondere einer ermittelten Restfeuchte des Behältnisses steuert.

Wie oben erwähnt, handelt es sich bei den Behältnissen um Behältnisse, welche aus Papier oder Pappe und insbesondere einer aus Papier oder Pappe gebildeten Pulpe aufgebaut ist.

Fig. 2 zeigt eine ähnliche Messanordnung, wobei jedoch hier mittels nah-infrarotem Licht gemessen wird. Die Behältnisse werden hier senkrecht zur Figurenebene transportiert. Auch bei dieser Art der Messung können diejenigen Bereiche, die eine höhere Restfeuchte aufweisen identifiziert werden, da hier die infrarote Strahlung stärker absorbiert wird.

Besonders bevorzugt erfolgt hier eine Messung mit zwei Spektralbereichen oder mehr Spektralbereichen, insbesondere um Umwelteinflüsse kompensieren zu können.

Fig. 3 zeigt eine Messung mittels thermischer Restfeuchtebestimmung. Hier wird ein Behältnis bereichsweise erwärmt und mit der Sensoreinrichtung 44 die Erwärmung erfasst. Dabei erfolgt eine Erwärmung der Behältnisse zu einem ersten Zeitpunkt t1 und bevorzugt erfolgt die Messung zu einem zweiten, etwas späteren Zeitpunkt t2. Bevorzugt wird die Temperatur der Behältnisse auch vor deren Erwärmung bestimmt. Es wäre jedoch auch möglich, eine Umgebungstemperatur zu bestimmen, welche annäherungsweise der Temperatur der Behältnisse vor deren Erwärmung entspricht.

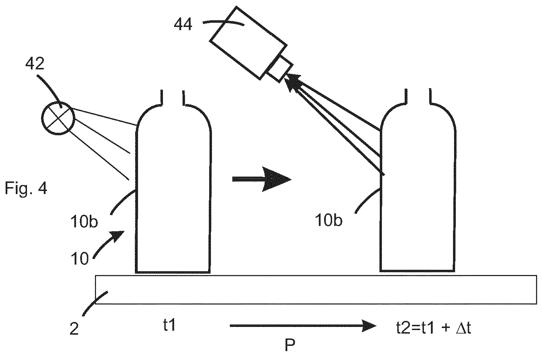

Fig. 4 zeigt eine Messung mittels thermischer Restfeuchtebestimmung. Hier wird ein Behältnis bereichsweise erwärmt und mit der Sensoreinrichtung 44 die Erwärmung erfasst. Dabei erfolgt eine Erwärmung der Behältnisse zu einem ersten Zeitpunkt t1 und bevorzugt erfolgt die Messung zu einem zweiten, etwas späteren Zeitpunkt t2. Bevorzugt wird die Temperatur der Behältnisse auch vor deren Erwärmung bestimmt. Es wäre jedoch auch möglich, eine Umgebungstemperatur zu bestimmen, welche näherungsweise der Temperatur der Behältnisse vor deren Erwärmung entspricht.

Die Anmelderin behält sich, vor sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Verfahren zum Bestimmen von Behältniseigenschaften, wobei ein zu untersuchendes Behältnis (10) entlang eines vorgegebenen Transportpfads transportiert wird und dieses Behältnis (10) einen Bodenbereich (10a), einen Grundkörper (10b) der zu Aufnahme eines Füllguts dient und einen Mündungsbereich (10c) aufweist und wobei dieses Behältnis wenigstens abschnittsweise aus einem Papier- oder Pappe enthaltenden Material besteht,
**dadurch gekennzeichnet, dass**
eine Strahlungseinrichtung (42) wenigstens einen Abschnitt des Behältnisses mit elektromagnetischer Strahlung bestrahlt und eine Sensoreinrichtung (44) von dem Behältnis in Reaktion auf die eingestrahlte Strahlung reflektierte und/oder transmittierte und/oder ausgestrahlte Strahlung erfasst und aus dieser Strahlung wenigstens eine für das Behältnis (10) charakteristische Größe ermittelt wird.

2. Verfahren nach Anspruch 1,
**dadurch gekennzeichnet, dass**
die eingestrahlte Strahlung aus einer Gruppe von elektromagnetischen Strahlungen ausgewählt ist, welche ultraviolette Strahlung, Strahlung im sichtbaren Wellenlängenbereich, Infrarote Strahlung; Wärmestrahlung, Terahertzstrahlung, Radiowellen, Mikrowellenstrahlung, Röntgenstrahlung, radioaktive Strahlung und dergleichen enthält.

3. Verfahren nach wenigstens einem der vorangegangenen Ansprüche,
**dadurch gekennzeichnet, dass**
die Strahlung infrarote Strahlung in einem Wellenlängenbereich zwischen 1200nm und 1800nm ist oder die Strahlung Wärmestrahlung ist, welche einen definierten Wärmeeintrag in wenigstens einem Abschnitt des Behältnisses bewirkt oder die Strahlung Röntgenstrahlung in einem Energiebereich zwischen 10keV und 30keV ist.

4. Verfahren nach wenigstens einem der vorangegangenen Ansprüche,
**dadurch gekennzeichnet, dass**
das Behältnis aus einer papier- oder pappehaltigen Pulpe besteht.

5. Verfahren nach wenigstens einem der vorangegangenen Ansprüche,
**dadurch gekennzeichnet, dass**
die Sensoreinrichtung ein ortsaufgelöstes Bild der von dem Behältnis reflektierten und/oder transmittierten und oder ausgestrahlten Strahlung aufnimmt.

6. Verfahren nach wenigstens einem der vorangegangenen Ansprüche,
**dadurch gekennzeichnet, dass**
die Messung der charakteristischen Größe während einer Bewegung der Behältnisse erfolgt.

7. Verfahren nach wenigstens einem der vorangegangenen Ansprüche,
**dadurch gekennzeichnet, dass**
die für das Behältnis (10) charakteristische Größe ein Feuchtewert und insbesondere Restfeuchte des Behältnisses und/oder eine Wanddicke wenigstens eines Wandungsabschnitts des Behältnisses (10) ist.

8. Verfahren nach wenigstens einem der vorangegangenen Ansprüche,
**dadurch gekennzeichnet, dass**
das Behältnis zum Zweck der Bestimmung der Behältniseigenschaften wenigstens zeitweise zwischen einer Strahlungseinrichtung (42) und der Sensoreinrichtung (44) angeordnet wird.

9. Verfahren nach wenigstens einem der vorangegangenen Ansprüche,
**dadurch gekennzeichnet, dass**
die wenigstens eine für das Behältnis (10) charakteristische Größe in der Längsrichtung (L) des Behältnisses abschnittsweise ermittelt wird und/oder die wenigstens eine für das Behältnis (10) charakteristische Größe in Abhängigkeit von der Längsrichtung des Behältnisses bestimmt wird.

10. Verfahren nach wenigstens einem der vorangegangenen Ansprüche,
**dadurch gekennzeichnet, dass**
das Behältnis während der Messung gedreht und insbesondere bezüglich seiner Längsrichtung gedreht wird.

11. Vorrichtung zum Bestimmen von Behältniseigenschaften, mit einer Transporteinrichtung (2) welche ein zu untersuchendes Behältnis (10) entlang eines vorgegebenen Transportpfads transportiert wobei dieses Behältnis (10) einen Bodenbereich (10a), einen Grundkörper (10b) der zu Aufnahme eines Füllguts dient und einen Mündungsbereich (10c) aufweist und wobei dieses Behältnis wenigstens abschnittsweise und bevorzugt vollständig aus einem Papier- und/oder Pappe enthaltenden Material besteht,
**dadurch gekennzeichnet, dass**
die Vorrichtung (1) eine Strahlungseinrichtung (42) aufweist, welche auf wenigstens einen Abschnitt des Behältnisses elektromagnetische Strahlung einstrahlt und eine Sensoreinrichtung (44), welche von dem Behältnis und/oder diesem Abschnitt in Reaktion auf die eingestrahlte Strahlung reflektierte und/oder transmittierte und/oder ausgestrahlte Strahlung erfasst und weiterhin eine Auswerteeinrichtung vorgesehen ist, welche aus dieser Strahlung wenigstens eine für das Behältnis (10) charakteristische Größe ermittelt.

12. Vorrichtung (1) nach dem vorangegangenen Anspruch,
**dadurch gekennzeichnet, dass**
die Sensoreinrichtung eine Bildaufnahmeeinrichtung aufweist, welche zur Aufnahme eines ortsaufgelösten Bildes wenigstens eines Abschnitts des Behältnisses ausgebildet und angeordnet ist wobei bevorzugt die Bildaufnahmeeinrichtung aus einer Gruppe von Bildaufnahmeeinrichtungen ausgewählt ist, welche Röntgenkameras, insbesondere Röntgen Zeilen- oder Matrixkameras, ortsauflösende Nahinfrarot - Kameras, ortsauflösende SWIR Kameras, Arrays mit Einzelsensoren und dergleichen enthält.

13. Vorrichtung nach wenigstens einem der vorangegangenen Ansprüche,
**dadurch gekennzeichnet, dass**
die Transporteinrichtung (2), die Strahlungseinrichtung (42) und die Sensoreinrichtung (44) derart angeordnet sind, dass sich das Behältnis wenigstens zeitweise zwischen der Strahlungseinrichtung (42) und der Sensoreinrichtung (44) befindet.

14. Vorrichtung (1) nach wenigstens einem der vorangegangenen Ansprüche,
**dadurch gekennzeichnet, dass**
die Vorrichtung (1) eine Dreheinrichtung aufweist, welche dazu geeignet und bestimmt ist, die Behältnisse bezüglich ihrer Längsrichtung zu drehen.

15. Anordnung zum Behandeln von Behältnissen mit einer Vorrichtung (1) nach wenigstens einem der vorangegangenen Ansprüche und mit einer Trocknungseinrichtung zum Trocknen der Behältnisse, wobei die Trocknungseinrichtung in Abhängigkeit von der für das Behältnis charakteristischen Größe steuerbar ist.
